# EUROPEAN PATENT APPLICATION

(11) **EP 1 532 982 A2**
(43) Date of publication of application: **25.05.2005**
(21) Application number: 04386026.1
(22) Date of filing: 12.10.2004
(51) Int. Cl.: A61K 35/78, A61K 33/22, A61P 17/00

(54) **Pharmaceutical ointment for the complete therapy of dermatologic diseases**

(30) Priority: 15.10.2003 GR 2003100419
(71) Applicant: Kapaj, Thelleza Skender, 115 26 Ampelokipi, Athens (GR)
(72) Inventor: Kapaj, Thelleza Skender, 115 26 Ampelokipi, Athens (GR)

(57) **Abstract**

Pharmaceutical ointment for use in the therapy of dermatologic diseases consisting of Vaseline as excipient (base), Hydrochloric Acid solution 37%, Boric Acid and Oil of Black Cedar. It cures completely all types of eczemas, hemorrhoids, fungi, trichophyton, acne, cutaneous allergies, condylomas, burns and certain types of lupus and psoriasis. It is effective in almost every type of dermatologic disease without any side effects.

The curing period varies from two weeks to 9 months depending on the case. The disease never reappears.

The ointment should be kept in a cool place in a glass or porcelain vessel.

## Description

The present invention relates to a new ointment, the application of which accomplishes complete cure of various types of eczema (inherited eczema, from stress, caused by chemical substances, etc.), fungi, allergies, trichophyton condylomas, hemorrhoids, acne, lupus and psoriasis.

### Prior Art

The recent commercially available pharmaceutical preparations do not provide effective therapy for the above mentioned dermatopathies. Many of them contain cortisone as the active ingredient. Additionally, none of them obtains a complete cure of the dermatologic disease. Some time after the completion of the treatment the disease usually reappears.

In the literature there are references to various preparations which are based on traditional methods and use extracts of some plants considered to have therapeutical effects.
In patent US5955081 a liquid extracted from the roots of the plant Asphodelus Microcarpus is mixed with acetic acid and the composition is used for the treatment of Psoriasis by applying it to the affected area of the skin at an appropriate frequency.
In patent EP0995443 the juice obtained from the roots of Asphodelus Albus is used to the preparation of a medicament for the treatment of Herpes, grazes and burns.
In the article of Ali-Shtayeh, Suheil Abu Ghdeib, "Antifungal activity of plant extracts against dermatophytes", Mycoses 42,665-672, 1999, the aqueous extracts of 22 plants used in folkloric medicine were investigated for their antifungal activity. Among them one can distinguish Asphodelus luteus and Asphodelus arvensis whose roots were used in some cases and the whole plant in others.

In all these patents and articles the activity of the medicament obtained is limited to a very small number of cutaneous diseases.
In our previous patent application EP1114643 we disclosed an ointment consisting of Vaseline, oil of black cedar, Boric acid (H₃BO₃), solution of Hydrochloric acid (HCl) and extract of the roots of the plant Asphodelus Lutea. That ointment was very effective in the treatment of many dermatologic diseases like eczema, fungi, allergies, trichophyton condylomas, hemorrhoids, lupus and psoriasis.
However, after many years of application of this ointment in the therapy of the above mentioned diseases we came to the conclusion that although the extract of the plant Asphodelus relieves and softens the affected area of the skin, it does not contribute to its therapy. The main active ingredient is the solution of Hydrochloric acid which acts in conjunction with the other ingredients present in the ointment to cure the disease. For this reason we have removed the extract of the plant Asphodelus from the composition of the ointment. We were also able to determine after many studies and trials the exact quantitative composition of the ointment for each application (dermatologic disease to be treated) in order to maximise its therapeutic action.

The advantage of the ointment disclosed in the present application is its ability to cure completely and permanently a large number of cutaneous diseases. After completion of the treatment the disease will never reappear.

### Synthesis and method of production

As a base (excipient) for the preparation of the ointment we use Vaseline.
For every 1000 gr of Vaseline we add:
1. 100-150 ml solution of Hydrochloric Acid Fuming 37%.
2. 30 gr of Boric Acid.
3. 30 gr Oil of Black Cedar

The above mentioned four ingredients are put in an appropriate vessel and are mixed well until a homogeneous ointment is formed.
It is subsequently stored in a glass vessel or a vessel made of porcelain, but never in a plastic or rubber vessel.

### Uses of the pharmaceutical ointment

The pharmaceutical ointment of the present invention is very effective in the therapy of the following diseases:
a. Eczemas (inherited eczema, from stress, caused by chemical substances, etc.)
b. Fungi
c. Cutaneous allergies
d. Trichophyton
e. Condylomas
f. Hemorrhoids
g. Acne
h. Certain types of Lupus
i. Certain types of Psoriasis
j. burns

The proportion of the Hydrochloric Acid solution in the ointment varies depending on the disease to be treated. More precisely:

In the treatment of Eczemas, Fungi, Lupus, Acne, and allergies the ointment must contain from 100 to 145ml of HCl solution 37% in every 1000gr of Vaseline.
In the treatment of Trichophyton, Condylomas, and Hemorrhoids the ointment must contain from 100 to 125ml of HCl solution 37% in every 1000gr of Vaseline.
In the treatment of Psoriasis the ointment must contain from 135 to 150ml of HCl solution 37% in every 1000gr of Vaseline.
When there are open injuries on the skin the proportion of HCl solution in the ointment must be near the lower limit of 100 ml per 1000gr of Vaseline.

### Forms of Application

The effect of the ointment on the eczema is tranquillizing and stops itching and pain. After a period of time from the application of the ointment to the skin, small drops of water appear on the surface of the skin.

The ointment is used three times daily and as often as itching appears. It is spread gently onto the affected skin surface with the fingertips. When there are splits or open injuries in the skin, the ointment has to be applied around those open injuries, not in them, in order to avoid irritation. In case of washing, the ointment must be applied ten minutes after that.

Skin with eczemas must not be washed more than 2-3 times weekly, because water is irritating the skin, thus prolonging the period of therapy.

The complete therapy of eczema, lupus and psoriasis lasts 3-9 months, while for the other dermatologic diseases the therapy lasts from two weeks to three months.

If the patient does not follow the therapy correctly, there might occur irritation. In that case the application of the ointment should be reduced for a short period.

### Example of Eczema treatment

A 10 years old patient, suffering from eczema on the neck and lips had tried many pharmaceutical ointments without satisfactory results. She started using the ointment disclosed in the present invention on May 2001 and by December 2001 she was completely cured without any eczema traces left on the skin. Until today the eczema has not reappeared.

### Example of Hemorrhoids treatment

A 50 year old patient suffered from Hemorrhoids of 3^{rd} degree. By applying the present ointment twice a day (morning-evening) he was completely cured in 15 days.

### Example of Psoriasis treatment

A 30 year old patient suffering from psoriasis on her hands for 10 years was completely cured in 9 months. In her case no other treatment had such an effect. Until today the disease has not reappeared.

## Claims

1. Pharmaceutical ointment for use in the therapy of dermatologic diseases consisting of Vaseline, Hydrochloric Acid solution 37%, Boric Acid and Oil of Black Cedar in the following proportion:
- 1000gr of Vaseline used as an excipient
- 100 - 150ml of Hydrochloric Acid solution 37%
- 30gr of Boric Acid
- 30gr Oil of Black Cedar

2. Pharmaceutical ointment according to claim 1, containing from 100 to 145ml of Hydrochloric Acid solution 37%, for use in the therapy of Eczemas.

3. Pharmaceutical ointment according to claim 1, containing from 100 to 120ml of Hydrochloric Acid solution 37%, for use in the therapy of Fungi.

4. Pharmaceutical ointment according to claim 1, containing from 100 to 110ml of Hydrochloric Acid solution 37%, for use in the therapy of Trichophyton.

5. Pharmaceutical ointment according to claim 1, containing from 100 to 120ml of Hydrochloric Acid solution 37%, for use in the therapy of Condylomas.

6. Pharmaceutical ointment according to claim 1, containing from 100 to 125ml of Hydrochloric Acid solution 37%, for use in the therapy of Hemorrhoids.

7. Pharmaceutical ointment according to claim 1, containing from 100 to 130ml of Hydrochloric Acid solution 37%, for use in the therapy of Acne.

8. Pharmaceutical ointment according to claim 1 , containing from 100 to 140ml of Hydrochloric Acid solution 37%, for use in the therapy of certain types of Lupus.

9. Pharmaceutical ointment according to claim 1, containing from 135 to 150ml of Hydrochloric Acid solution 37%, for use in the therapy of certain types of Psoriasis.

10. Pharmaceutical ointment according to claim 1, containing from 100 to 120ml of Hydrochloric Acid solution 37%, for use in the therapy of cutaneous Allergies.
